# NEW EUROPEAN PATENT SPECIFICATION

(11) **EP 0 401 056 B2**
(45) Date of publication and mention of the opposition decision: **13.06.2007**
(45) Mention of the grant of the patent: 12.11.1997
(21) Application number: 90306062.2
(22) Date of filing: 04.06.1990
(51) Int. Cl.: A61K 31/195

(54) **Glutamine in the treatment of impaired host defences**
Glutamin zur Behandlung von beeinträchtigten Abwehrkräften
Glutamine dans le traitement de la diminution des défenses de l'hôte

(30) Priority: 02.06.1989 US 360839
(43) Date of publication of application: 05.12.1990
(73) Proprietor: THE BRIGHAM AND WOMEN'S HOSPITAL, INC., Boston, MA 02115 (US)
(72) Inventor: Smith, Robert J., Brookline, Massachusetts 02146 (US); Wilmore, Douglas W., Brookline, Massachusetts 02146 (US)
(74) Representative: Wibbelmann, Jobst

(56) References cited:
- WO-A1-87/01589
- M.S.M. Ardawi and E.A. Newsholme: Glutamine Metabolism in Lymphoid Tissues; D. Häussinger, H. Sies (Ed.), Springer Verlag (1984)
- Essays in Biochemistry, vol. 21 (1985), pp. 1-43
- Biochem. J., vol. 212 (1983), pp. 835-842
- J. Cell. Physiol., vol. 95 (1978), pp. 41-48
- Surgery, vol. 104, no. 5 (1988), pp. 917-923
- Annals of Allergy, vol. 59(5) (1987), pp. 17-20

## Description

The present invention relates to the use of glutamine (GLN) and functional analogues, derivatives, substitution products, isomers, or homologues of GLN which retain the characteristics of GLN, in connection with total parenteral nutrition (TPN) therapy in the preparation of a medicament for use in increasing lymphocyte proliferation in response to an antigen or a mitogen, antibody responses, or all mediated immune responses, wherein the activity of the immune system is decreased in an animal as a result of undergoing, or having been treated by bone marrow transplantation.

In patients undergoing bone marrow transplantation, for example, the radiation and/or chemotherapeutic regimens pre- and post-transplant, coupled with the frequent occurrence of graft-versus-host disease, produce both catabolic and serious nutritional consequences. Many factors contribe to a requirement for total parenteral nutrition (TPN) in these patients, including oral and esophageal mucositis, diarrhea, nausea, vomiting, xerostomia, and dysgeusia (Cunninhgam et al., Nurs. Clin. N. Amer., 18:585-596 (1983); Cheney et al., Cancer 59:1515-1519 (1987)). Although TPN is beleived to be necessary and beneficial in bone marrow transplant patients, trials with i.v. infusion of specialized formulas high in branched-chain amino acids (BCAA) during the first month after transplantation did not improve nitrogen balance (Lenssen et al., J. Parent. Ent. Nutr. 11:112-118 (1987)).

A compromised immune function brought on by the various causes listed above can be a major cause of death and disability. These clinical states are frequently associated with abnormal metabolism of the non-essential amino acid, glutamine (GLN). GLN can be synthesized to some extent by most tissues. Unlike most amino acids, GLN has two amine moieties: an alpha-amino group and an amide group. The presence of the amide group enables GLN to remove ammonia from the peripheral tissues of the body and transport nitrogen to visceral organs. In addition, it is common for tissues that remove GLN from the circulation to utilize the carbon skeleton for energy.

Glutaminase and glutamine synthetase are the two principal enzymes involved in the regulation of GLN metabolism. Glutaminase catalyzes the hydrolysis of GLN to glutamate and ammonia, while glutamine synthetase catalyzes the synthesis of GLN from glutamate and ammonia. While most tissues have both of these enzymes, usually one is more active than the other, depending on the particular tissue.

GLN synthesis and exportation occurs primarily in skeletal muscle and the brain. In turn, GLN is consumed by such replicating cells as fibroblasts, hemopoietic cells, lymphocytes, intestinal epithelium, and tumor cells. Characteristically, these cells possess high levels of glutaminase activity and low levels of intracellular GLN. This fact may have clinical significance for patients having large wounds, inflammation associated with infection, or a gastrointestinal dysfunction which precludes normal enteral feeding since the desirable proliferation of the cell types in these conditions may depend on the availability of sufficient levels of GLN.

In the gastrointestinal tract, GLN is used as a respiratory fuel. The enteral administration of GLN results in increased uptake of luminal GLN by the gut mucosa accompanied by a simultaneous decrease in uptake of GLN from the circulation. Thus, the consumption of GLN by the gut is balanced between these two sources of GLN.

Most of the GLN taken up by the gastrointestinal tract occurs via the epithelial cells lining the villi of the small intestine. The GLN metabolism which occurs in the small intestine provides a major source of energy for the gut and produces precursors for hepatic ureagenesis and gluconeo-genesis by processing nitrogen and carbon from other tissues.

Baskerville et al. (Brit. J. Exp. Pathol., 61:132 (1980)) lowered the concentration of plasma GLN to undetectable levels by infusing purified glutaminase into rhesus monkeys, marmosets, rabbits, and mice, resulting in vomiting, diarrhea, villus atrophy, mucosal ulcerations, and intestinal necrosis.

Martin et al. (U.S. Patent 2,283,817) disclose a composition containing GLN which is used as a detoxicant, rather than a dietary supplement. In the patent, GLN is combined synergistically with other amino acids to act directly on a toxin to inhibit any deleterious effect.

In Shive et al. (U.S. Patent 2,868,693), GLN-containing compositions for the treatment of peptic ulcers are disclosed.

Further evidence of the potential protective effect of GLN was shown by Okabe et al., Digestive Disease, 20:66 (1975), who found that GLN could protect against aspirin-induced gastric ulcerations in humans. These effects were not observed when GLN was given by tube feedings into the intestine, thus preventing direct exposure of the gastric mucosa to GLN. This indicates that the GLN effects on gastric ulceration were local and not secondary to altered systemic nutrition.

The non-essential amino acid GLN is not present in standard parenteral alimentation solutions yet is a preferred oxidative fuel for the small intestine (Windmeuller, H.G., Adv. Enzyml. 53:201-237 (1982)) and has trophic effects on the intestinal mucosa of intravenously (i.v.) fed rodents (Hwang, T.L. et al., Surgical Forum 37:56-58 (1986); O'Dwyer, S.T et al., Clin. Res. 35:369A (1987)). This visceral GLN requirement may be even greater during critical illness, when GLN metabolism by the small intestine is known to be increased (Souba et al., Surgery, 94(2):342 (1983)). Although GLN is known to be rapidly consumed by the pancreas and appears to be concentrated in the exocrine part of the gland (Cassano, G.B. et al., J. Neurochemistry 12:851-855 (1965)), the effect of exogenously administered GLN on the exocrine pancreas of intact animals has not been reported.

It is known from WO 87/01589 how to treat a catabolic dysfunction in an animal, which comprises administering to an animal a therapeutically effective amount of glutamine or an analogue thereof. A catabolic dysfunction results in the breakdown of anatomical structures. Such conditions can result in, but do not necessarily lead to reduced immune system activity. In addition, there are many conditions which result in reduced immune system activity where there is no catabolic tissue breakdown. Thus, the usefulness of glutamine in treatment of catabolic dysfunction may not include the usefulness in increasing the activity of the immune system.

The term "total parenteral nutrition" (TPN) is used to describe formulas for use in patients who derive their entire dietary requirements i.v. Total parenteral nutrition formulas, unlike enteral formulas, do not normally contain GLN. The absence of GLN from parenteral formulas is due, in part, to concern with respect to its instability at room temperature, and the resulting generation of ammonia and pyroglutamic acid. There has also been concern about the generation of glutamic acid from GLN because of the potential toxicity of glutamic acid as a neurotransmitter. In fact, these concerns do not appear to be justified at the pH values of enteral and parenteral nutrition solutions.

TPN results in villus atrophy, a phenomenon which is generally reversible when oral feedings are resumed. Since TPN formulas lack GLN, the body's requirements for this amino acid must be met from synthetic pathways in body tissues.

None of the prior art studies have shown that breakdown of the gut wall is leading to enhanced permeability, comprised immune function, which occur during TPN can be prevented through the administration of high levels of GLN.

The invention is set forth in more detail in the attached claims.

In the invention, an animal having, compromised immune function, following bone marrow transplantation, is given a therapeutically effective amount of GLN. This amount of GLN is greater than that normally encountered in the diet of healthy individuals.

Provision of GLN to patients undergoing bone marrow transplantation is especially beneficial in ameliorating compromised immune function consequent to TPN.

Preferred embodiments of the invention will now be described, with reference to the accompanying drawing, in which:

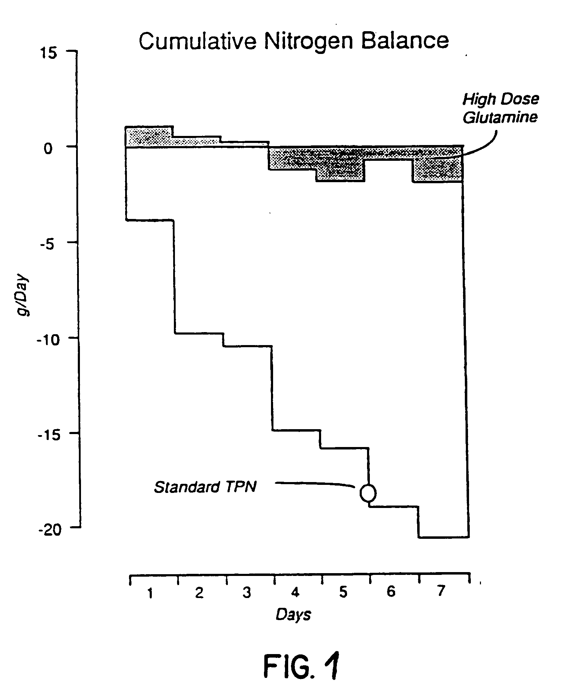

Figure 1 is a graphic presentation of data demonstrating the effect of high dose GLN TPN on cumulative nitrogen balance after allogeneic bone marrow transplantation.

The inventors have devised the manufacture of a medicament for treating compromised immune function consequent to TPN according to claim 1. The present invention comprises the administration to an animal afflicted with, or likely to develop, the above disease state, a therapeutically effective amount of GLN or a functional analogue thereof. This therapeutically effective amount is greater than that present in a normal diet. The normal dietary intake for humans is about 2 to 4 g/day.

The term "enteral" is intended to indicate a method of administration of nutrients to that portion of the alimentary canal between the stomach and the anus.

The term "parenteral" denotes method of administration of nutrients to that region outside of the digestive tract.

Animals in which the invention is effective are those commonly classified as mammals, including humans.

By the term "treating" is intended prevention, amelioration, or cure of a symptom or set of symptoms constituting a disease state.

The term "compromised immune function" denotes a reduction in an immunological function, which can be measured in vitro or in vivo. Such function consist of lymphocyte proliferation in response to an antigen or a mitogen, antibody responses, cell mediated immune responses such as delayed hypersensitivity. Various forms of compromised immune function, all of which are intended by the term, can lead to decreased host defenses to a variety of pathogens such as pathogenic microorganisms. Compromised immune function is a well-known consequence of treatment with chemotherapeutic drugs, and is also a consequence of certain forms of physical trauma, burns, malnutrition, diabetes and other disease states.

The term "impairment of host defense" denotes an increase in susceptibility to infection by a variety of pathogenic microorganisms, including, but not limited to, bacteria, viruses, and fungi. Such impairment occurs following breakdown in the gut wall barrier, allowing the normally prevented passage of pathogens from the gut lumen across the mucosal barrier. Such impairment may result from compromised immune function, in which the cells of the immune system are suppressed in their ability to respond to the antigenic structures associated with the pathogens. Impaired host defense also results from abnormal function of other cells including depressed leukocyce chemotaxis, phagocytosis, and killing, altered macrophage function, and of other innate mechanisms of host resistance, which are well known in the art (see Mims, C.A., The Pathogenesis of Innfectious Disease (2nd Edition), 1982, Academic Press, N.Y)

A requirement for GLN in tissue culture medium for the survival or growth of a large variety of cell types is well known in the art (Freshney, CULTURE OF ANIMAL CELLS, A Manual of Basic Technique, Alan R. Liss, Inc., New York, 1983). It is well known, for example, that lymphocyte proliferation and differentiation in vitro is highly dependent on increased concentrations of GLN in the medium. Insufficiency of GLN in vivo, for example following TPN to which GLN has not been added, can be shown to lead to a suppression of lymphocyte function. This loss of function is either prevented in part, or is partially restored, by the addition of GLN to the TPN formula.

The term "substantially associated with," as applied to the dysfunctions or symptoms for which the method of the invention is effective, means those dysfunctions wherein the biochemical demand for GLN occurs during or after the dysfunction, and is related thereto.

The administration of GLN can be by both enteral and parenteral means.

Examples of the means of enteral administration of GLN is the use of small-bore tubing placed via the nose into the gastric or duodenal regions, or through surgical implantation as in, for example, gastrostomy, or jejunostomy.

Examples of parenteral routes of administration include, but are not limited to, subcutaneous, intramuscular or intravenous injection, and nasopharyngeal, mucosal or transdermal absorption. In most cases, the GLN is administered i.v. In i.v. administration, the therapeutically effective amount of GLN, in liquid form, is directly administered from a reservoir from which tubing connects to a needle which is placed into a large vein of the patient.

Regardless of which route of administration is utilized, the GLN can be administered either singly or as a dietary supplement. When used as a dietary supplement, the GLN can be mixed with an existing enteral or parenteral diet prior to administration to the patient. It is also possible to administer the GLN without mixing it directly with the other components of a diet as, for example, in i.v. feeding wherein the GLN is not directly added to the main i.v. bottle, but instead is added to a common reservoir using a "piggy-back" bottle.

Functional analogues, derivatives, substitution products, isomers, or homologues of GLN which retain the characteristics of GLN are contemplated as equivalents. Preferred are those analogues capable of donating an amine group and being metabolized in the Krebs cycle. Most preferred are compounds which possess the amino acid residue at one terminus of a carbon chain and an amine moiety at the other terminus of the carbon chain.

The therapeutically effective dose ranges for the administration of GLN are those large enough to prevent the catabolism or atrophy of the tissues of the body in order to maintain metabolic homeostasis. In an enteral diet GLN would be administered at a rate greater than or equal to 0.3 grams per kilogram of body weight per day. Such administration rates could be 0.3 to 2.0 grams per kilogram of body weight per day, preferably 0.3 to 1.5 grams per kilogram of body weight per day, and more preferably 0.4 to 1.0 grams per kilogram of body weight per day. The rate of administration for GLN when administered i.v. would be greater than or equal to 0.1 grams per kilogram of body weight per day. Such administration rates could be 0.2 to 3.0 grams per kilogram of body weight per day, preferably 0.3 to 2.5 grams per kilogram of body weight per day, more preferably 0.4 to 2.0 grams per kilogram of body weight per day.

According to the invention, GLN may be administered by simply modifying existing dietary formulas to contain the proper concentration of GLN. Most preferably, the GLN would remain in a dry form such as, for example, a sterile lyophilized powder which is aseptically hydrated at the time of administration and mixed at the proper concentration with the other components of the dietary composition. Alternatively, the GLN could be premixed with the other components of a dry formula which is aseptically rehydrated at time of administration, or stored as a frozen concentrate which is thawed and mixed at the proper concentration at time of use.

The use of GLN by the method according to the invention is ideally suited for the preparation of compositions. These compositions may comprise GLN, GLN-containing dipeptides, GLN salts, either alone or in combination with other chemicals. These other chemicals can be pharmaceutically acceptable carriers, as well as other active substances of the diet as, for example, free amino acids, protein hydrolysates, or oils.

Preparations for parenteral administration include sterile aqueous or non-aqueous solutions, suspensions, and emulsions. Carriers or occlusive dressings can be used to increase skin permeability and enhance cutaneous absorption.

Containers containing the composition of the invention can be used to facilitate the administration of GLN according to the method of the invention. These containers are designed to contain, for example, the daily dosage of GLN to be administered to the patient.

Containers adapted for i.v. administration of GLN alone or in combination with other amino acids are especially useful. Such containers could comprise a receptacle for the liquid GLN-containing composition, and a liquid conducive means capable of attachment to a needle.

The liquid conducive means could be any object capable of conveying the liquid composition in the receptacle to the needle such as, for example, plastic tubing.

The needle attached to the conducive means could either be inserted directly into a blood vessel or i.v. catheter of the human recipient or could be inserted into a reservoir to enable mixing with another solution before being administered to the patient.

The above disclosure generally describes the present invention. A more complete understanding can be obtained by reference to the following specific examples which are provided herein for purposes of illustration only.

### EXAMPLE 1

### Effect of Glutamine-Enriched Parenteral Nutrition Following Radiation, Chemotherapy, and Bone Marrow Transplantation

This study was performed to determine in a randomized, blinded clinical trial whether the provision of dietary GLN to patients undergoing total body irradiation and/or chemotherapy would alter nitrogen balance, improve clinical outcome and accelerate general recovery.

The study population included patients already enrolled in the autologous and allogeneic bone marrow transplant protocols.

### A. MATERIALS AND METHODS

### 1. Patients

Patients were recruited from those already enrolled in autologous or allogenic bone marrow transplant treatment protocols. Under these protocols eligible patients are between the ages of 18 and 60 and of either sex, diagnosed with acute myelogenous leukemia (AML) in first remission (allogenic transplant), AML in second remission (autologous transplant), myelodysplasyic syndromes, lymphoma, aplastic anemia and other leukemias including chronic myelogenous leukemia (CML). The patients in this protocol required parenteral nutrition and had a dedicated central line for infusion. They had normal hepatic function (total bilirubin < 3 mg/dl), normal renal function (creatinine < 1.2 mg/dl), absence of diabetes (blood glucose < 200 mg/dl without insulin or oral hypoglycemic agents). They were in the stable phase of their disease (e.g., in remission). Patients had not lost more than 20% of body weight below their ideal body weight.

### 2. Study Design

Patients were randomized into one of two groups: standard or GLN containing TPN. The diets were isonitrogenous and isocaloric but contained 0 GLN in the standard formula and 0.57 g GLN/kg body weight/day in the experimental solution. The patient, bone marrow transplant physicians and the investigators who see the patients were blinded as to treatment group. Under this design, the electrolyte requirements for individuals may vary over time depending on the clinical conditions and the physician may vary the electrolyte content of the solution depending on blood levels and clinical status in consultation with the investigators and Nutrition Support Service.

Patients took oral nutrient ad libitum throughout the study if allowed by their primary physicians. Daily oral calorie, protein, fat and carbohydrate intake was recorded.

Administration of the blinded test solution was initiated within five days after the day of bone marrow transplantation (referred to as day 0). After an initial equilibration period of 3 days, collection of all urine and stool began each 24-hours for the next seven days. No collections were performed the next seven days but resumed for the subsequent 7 days (i.e., approximately the third week post-transplant). Collections were from 4 p.m. to 4 p.m., corresponding to the time when the solution bags are hung and administered by the 9D nursing staff.

Blood was drawn weekly for GLN, glutamate, ammonia, amino acids, C-reactive protein and prealbumin. Other blood samples were obtained by the primary care physician for monitoring TPN and organ function

The patients were randomized to treatment or control groups by unblinded research pharmacists. The groups were balanced for diagnosis and within the AML group in 1 st remission, the groups were also balanced for treatment (with or without total body irradiation).

The study was terminated when the patient no longer required TPN as indicated by three consecutive days of ad libitum oral intake which averages 50% of calorie requirements. The third day of 50% oral intake is defined as the end of TPN. In all cases, the patients were included in the final outcome analysis only if they received at least 60% of required calories and protein of the blinded solution during the time period when TPN was required. The protocol calls for patients to be dropped from the study if 5 consecutive days pass during which he receives none of the blinded solution.

### 3. Nutritional Intake

Calorie intake was set at approximately 1.5 times estimated metabolic rate (based on height and weight. The non-protein calorie intake was approximately 70% dextrose and 30% fat emulsion. The protein intake was 1.5 g/kg/day. Electrolytes and minerals were added in adequate amounts to maintain blood concentrations within the normal range. Vitamins were added as MVI-12 10 ml/24 h. Trace element solution were added as Lypho-med^{®} 1 ml/24 h. The TPN solution was administered on a 4 p.m. to 4 p.m. schedule by the ward nurses. In patients exceeding 20% of ideal body weight, the calorie and protein requirements were based on ideal weight for age and sex.

### 4. Medication

All medication and non-TPN fluids were given to these patients as deemed necessary by their physicians. All drugs and fluids which the patients received were recorded.

### 5. Additional Methods of Evaluation and End Points

Patients were evaluated for body composition (pre- and post-TPN), mid-arm muscle mass (pre and post treatment), hand grip strength (pre, days 7 and 21, and post-TPN), lactulose permeability (days 7 and 21, and post-TPN), sepsis severity score (weekly), graft vs. host disease, mcositis score (three times a week), general assessment of well-being (pre- and post-TPN).

The major end points evaluated included: (1) Nitrogen balance (week 1 and week 3); (2) Excretion of 3-methyl histidine and creatinine (mean of 3 urine values collected during days 5-7 of week 1 and week 3); (3) CRP (averaged over the first 4-6 weeks of TPN); (4) Body composition; (5) Mean weekly sepsis score; (6) Mean weekly fever and weekly mean of daily maximal temperature; and (7) Mean weekly GVH grade

The minor end points evaluated include: (1) Days in unit (from time of transplant, T = 0); (2) Days from transplant until recontamination; (3) Hospital charges, total antibiotic charges; (4) Antibiotic(s) ordered times the number of days administered, i.e., antibiotic days--only while on TPN and only counting those antibiotics added to the patient's treatment for known or suspected infection; (5) Days until leukocyte counts return to >500 and >1,000 cells/cm³; (6) Days from transplant until absolute neutrophil count returns to >500 cells/cm³; (7) Lactulose permeability, measured on two occasions at the end of two balance study periods; (8) Units of transfused platelets and red blood cells; (9) Days on TPN until 50% oral intake or discharge; and (10) Feeling of well-being.

### B. RESULTS

Results obtained with 2 patients are shown in Figure 1 and Table I. TPN with high dose GLN induced a marked improvement in the clinical status of the transplant patients. This treatment prevented the increasingly negative nitrogen balance, decreased the severity of oral mucositis (largely induced by the chemotherapy), and improved the patients general health as assessed by the 3 clinical criteria shown. It is concluded that GLN has a palliative effect on the health of this class of severely ill patient.

**Table I**

| EFFECT OF GLUTAMINE- SUPPLEMENTED TPN FOLLOWING BONE MARROW TRANSPLANTATION | | |
|---|---|---|
| | Standard TPN | High Dose GLN |
| Nitrogen Balance (g/day) | -2.9 | +0.6 |
| Antibiotic Days¹ | 60 | 43 |
| Days in Unit² | 46 | 39 |
| Days until Contamination³ | 36 | 28 |
| Mucositis Score⁴ | 2.5 | 1.0 |

| | | |
|---|---|---|
| ¹ Product of the number of additional antibiotics added to the standard regimen and the number of days the patient is kept on the antibiotic ² Number of days patients are kept in Protective Environment (laminar air-flow rooms) until a clinical decision is made to release them (criteria include WBC > 1000, no fever, readiness to eat by mouth, etc.) ³ Number of days patient is kept germ-free until physical contact with "contaminated" individual (usually a family member) is allowed. This is also a clinical decision. ⁴ Clinical grading of oral mucositis on a scale of 0 to 4 of increasing severity) | | |

### EXAMPLE 2

### [This example does not fall within the scope of the claims] Glutamine-enriched Parenteral Nutrition Suppresses Damage to Gut Wall Induced by 5-Fluorouracil

Lactulose is a non-metabolizable sugar that is poorly absorbed from the gastrointestinal tract. In gastrointestinal disorders such as inflammatory bowel disease, lactulose is taken up through gaps in the mucosal membrane, allowing its excretion in the urine. The amount of urinary lactulose is therefore a measure of "leakage" across the gut mucosal barrier (Menzies, Biochem. Soc. Trans. 550:1042-1047 (1974)).

Rats were treated with 5-fluorouracil as described in Example 8, above. Lactulose, 2 ml iso-osmolar solution of 100 mg/ml, was infused by gavage and urine was collected over the subsequent 24 hr and frozen. Urinary lactulose was determined enzymatically as described by Ziegler et al. (Arch. Surg. 123:1313-1319 (1988)). Treatment with a single injection 5-FU was found to increase gut permeability to lactulose by 24 h. This increase peaked at between 24 and 48 h after chemotherapy. Over a three day period, animals receiving GLN-enriched TPN, had significantly reduced gut permeability compared to animals fed standard TPN. For example, at 48-72 h. after 5-FU, 24 h lactulose excretion was reduced from 32 to 17 µmoles in the GLN-treated rats.

It is concluded that GLN protects the gut wall from the increase in permeability that accompanies chemotherapeutic drugs. This effect is expected to be of major importance in preventing infection via the Gl route, and likely contributes to the more rapid recovery of patients undergoing bone marrow transplantation, as described in Example 1 above.

### EXAMPLE 3

### Glutamine Alleviates Immunosuppressive Effects of TPN

Rats were fed with either laboratory chow, TPN, or GLN-supplemented TPN.

The animals received either standard rat chow orally, a standard parenteral alimentation solution without GLN (Control) or a 2% GLN-enriched solution by constant i.v. infusion for 7 days.

The i.v. diets provided all nutrients essential for growing rats. The solutions were prepared and kept sterile. The parenteral diets were isocaloric (1.13 KcaUml), isonitrogenous (9.6mg/ml) and differed from one another only in the composition of non-essential amino acids.

Seven days after initiation of treatment, animals were sacrificed and the spleen, thymus, and mesenteric lymph nodes were harvested. Cell suspensions were prepared from these organs using standard techniques (see Klein, Immmunology: The Science of Self-Nonself Discrimination, Wiley-Interscience, 1982). Lymphocyte proliferative responses in vitro in the presence of mitogens were carried out using methods well known in the art. Compared to the chow group, TPN for 1 week markedly reduced lymphocyte responsiveness by as much as 50%. This suppression was reversed by the presence of GLN in the parenteral solution, such that the GLN treated rats showed lymphocyte responses intermediate between those of the TPN animals and the control chow-fed rats.

The presence of GLN in the parenteral solution therefore significantly alleviates the immunosuppressed state produced by TPN in the absence of GLN. The breakdown of the gut mucosal barrier, as described in Example 2 above, combines with the compromised immune function to increase the susceptibility to infection of the animal maintained on TPN. Enhanced susceptibility is likely not only to bacteria and other microorganisms entering via the gut, but by any other route as well (due to the compromised immune function). GLN-supplemented TPN is capable then of promoting host defense by several independent mechanisms. The improved infectious status of the patients undergoing bone marrow transplantation (see Example 1, above) serves as corroborative evidence for such "pro-host" activity of GLN.

## Claims

1. A use of glutamine (GLN) and functional analogues, derivatives, substitution products, isomers, or homologues of GLN which retain the characteristics of GLN, in connection with total parenteral nutrition (TPN) therapy, in the preparation of a medicament for use in increasing lymphocyte proliferation in response to an antigen or a mitogen, antibody responses, or all mediated immune responses, wherein the activity of the immune system is decreased in an animal as a result of undergoing, or having been treated by bone marrow transplant.

2. The use as claimed in claim 1 wherein the medicament is used in treating a compromised immune function, associated with cancer radiation therapy or chemotherapy.

3. The use as claimed in claim 1 or 2, wherein the medicament is for the treatment and to promote recovery in a human.

4. The use as claimed in any one of claims 1 to 3, wherein the medicament is adapted for parenteral administration.

5. The use as claimed in any one of claims 1 to 4, wherein the medicament is adapted to be administered at the rate of 0.2 to 3.0 grams per kilogram of body weight per day of GLN.

6. The use as claimed in anyone of claims 1 to 4, wherein the medicament is adapted to be administered at the rate of 0.3 to 2.5 grams per kilogram of body weight per day of GLN.

7. The use as claimed in any one of the claims 1 to 4, wherein the medicament is adapted to be administered at the rate of 0.4 to 2.0 grams per day of GLN.

## Patentansprüche

1. Verwendung von Glutamin (GLN) und funktionellen Analoga, Derivaten, Substitutionsprodukten, Isomeren oder Homologen von GLN, welche die Eigenschaften von GLN in Verbindung mit einer Therapie der vollständigen parenteralen Ernährung (TPN) beibehalten, bei der Herstellung eines Medikaments zur Verwendung bei der Steigerung der Lymphozyten-Proliferation als Reaktion auf ein Antigen oder ein Mitogen, Antikörperreaktionen, oder alle mittelbaren Immunreaktionen, bei denen die Aktivität des Immunsystems bei einem Tier als Folge davon verringert ist, dass es einer Knochenmarkstransplantation unterzogen wird oder damit behandelt worden ist.

2. Verwendung nach Anspruch 1, bei der das Medikament bei der Behandlung einer beeinträchtigten Immunfunktion, die mit einer Krebs-Bestrahlungstherapie oder -Chemotherapie einhergeht, verwendet wird.

3. Verwendung nach Anspruch 1 oder 2, bei der das Medikament zur Behandlung und zur Förderung der Genesung bei einem Menschen bestimmt ist.

4. Verwendung nach irgendeinem der Ansprüche 1 bis 3, bei der das Medikament zur parenteralen Verabreichung 30 vorgesehen ist.

5. Verwendung nach irgendeinem der Ansprüche 1 bis 4, bei der das Medikament dazu vorgesehen ist, dass es mit einer Rate von 0,2 bis 3,0 Gramm pro Kilogramm Körpergewicht pro Tag an GLN verabreicht wird.

6. Verwendung nach irgendeinem der Ansprüche 1 bis 4, bei
der das Medikament dazu vorgesehen ist, dass es mit einer Rate von 0,3 bis 2,5 Gramm pro Kilogramm Körpergewicht pro Tag an GLN verabreicht wird.

7. Verwendung nach irgendeinem der Ansprüche 1 bis 4, bei
der das Medikament dazu vorgesehen ist, dass es mit einer Rate von 0,4 bis 2,0 Gramm pro Tag an GLN verabreicht wird.

## Revendications

1. Utilisation de glutamine (GLN) et d'analogues, dérivés, produits de substitution, isomères ou homologues fonctionnels de la GLN qui conservent les caractéristiques de la GLN dans le cadre d'un traitement par nutrition parentérale totale (NPT) pour la préparation d'un médicament à utiliser pour accroître la prolifération des lymphocytes en réponse à un antigène ou un mitogène, les réponses d'anticorps, ou toutes les réponses immunitaires à médiation, où l'activité du système immunitaire est abaissée chez un animal parce qu'il subit une greffe de moelle osseuse ou a été traité par une greffe de moelle osseuse.

2. Utilisation selon la revendication 1, où le médicament est utilisé pour traiter une altération de la fonction immunitaire associée à la radiothérapie ou la chimiothérapie anticancéreuses.

3. Utilisation selon la revendication 1 ou 2, où le médicament est destiné au traitement et à l'accélération de la guérison chez l'homme.

4. Utilisation selon l'une quelconque des revendications 1 à 3, où le médicament est adapté pour l'administration parentérale.

5. Utilisation selon l'une quelconque des revendications 1 à 4, où le médicament est adapté pour être administré à raison de 0,2 à 3,0 g de GLN par kg de poids corporel par jour.

6. Utilisation selon l'une quelconque des revendications 1 à 4, où le médicament est adapté pour être administré à raison de 0,3 à 2,5 g de GLN par kg de poids corporel par jour.

7. Utilisation selon l'une quelconque des revendications 1 à 4, où le médicament est adapté pour être administré à raison de 0,4 à 2,0 g de GLN par jour.
